(19) Europäisches Patentamt / European Patent Office / Office européen des brevets

(11) **EP 3 770 272 B1**

(12) **EUROPEAN PATENT SPECIFICATION**

(45) Date of publication and mention of the grant of the patent:
**22.09.2021 Bulletin 2021/38**

(51) Int Cl.:
***C12Q 1/68*** *(2018.01)*     ***C12Q 1/6851*** *(2018.01)*
***G16B 45/00*** *(2019.01)*

(21) Application number: **20191740.8**

(22) Date of filing: **27.08.2014**

(54) **AN ANALYSIS METHOD AND SYSTEM FOR ANALYZING A NUCLEIC ACID AMPLIFICATION REACTION**

ANALYSEVERFAHREN UND SYSTEM ZUR ANALYSE EINER NUKLEINSÄUREAMPLIFIKATIONSREAKTION

PROCÉDÉ ET SYSTÈME D'ANALYSE PERMETTANT D'ANALYSER UNE RÉACTION D'AMPLIFICATION D'ACIDE NUCLÉIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.01.2021 Bulletin 2021/04**

(62) Document number(s) of the earlier application(s) in accordance with Art. 76 EPC:
**14182415.1 / 2 990 490**

(73) Proprietors:
• **Roche Diagnostics GmbH**
**68305 Mannheim (DE)**
• **F. Hoffmann-La Roche AG**
**4070 Basel (CH)**

(72) Inventor: **KNOBEL, Rolf**
**6343 Rotkreuz (CH)**

(74) Representative: **Richardt Patentanwälte PartG mbB**
**Wilhelmstraße 7**
**65185 Wiesbaden (DE)**

(56) References cited:
**EP-A1- 1 518 935**     **EP-A1- 2 719 770**
**US-A1- 2003 148 302**     **US-A1- 2008 154 511**
**US-A1- 2014 095 080**

• **THOMAS D SCHMITTGEN ET AL: "Analyzing real-time PCR data by the comparative CT method", NATURE PROTOCOLS, vol. 3, no. 6, 1 June 2008 (2008-06-01), pages 1101-1108, XP055137608, ISSN: 1754-2189, DOI: 10.1038/nprot.2008.73**
• **E. B. SHAIN ET AL: "A new method for robust quantitative and qualitative analysis of real-time PCR", NUCLEIC ACIDS RESEARCH, vol. 36, no. 14, 1 January 2008 (2008-01-01), pages e91-e91, XP055055640, ISSN: 0305-1048, DOI: 10.1093/nar/gkn408**

EP 3 770 272 B1

**Description**

**FIELD OF THE INVENTION**

The present invention relates to an analysis method for a real-time nucleic acid amplification reaction RNR such as a polymerase chain reaction PCR and a system for analyzing a nucleic acid amplification reaction of an analyte, such as for detecting a presence and/or measuring a quantity of an analyte in a sample by a nucleic acid amplification reaction.

**BACKGROUND AND RELATED ART**

In vitro nucleic acid amplification techniques include a variety of methods based on different approaches. One approach includes methods by which a DNA or RNA sequence is multiplied, thus making it more readily detectable for various procedures or tests. For example, in vitro amplification of the nucleic acid can be done using one of the following methods: Polymerase chain reaction PCR, Ligase chain reaction (LCR), or isothermal transcription mediated amplification (TMA) method. In all of the above mentioned approaches the nucleic acid is subjected to repetitive amplification cycles.
A technique that is frequently used for various medical, biological or industrial applications is the PCR technique. This technique relies on thermal cycling, comprising cycles of repeated heating and cooling for enzymatic replication of the nucleic acid in order to amplify a specific region of the nucleic acid strand. As PCR progresses, the DNA generated is itself used as a template for replication, setting in motion a chain reaction in which the DNA template is exponentially amplified doubling (at least theoretically) with each reaction cycle.
Nucleic acid amplification techniques include many variations. One of these variations is called real-time nucleic acid RNR and is based on amplifying and simultaneously detecting the presence of nucleic acid in the sample and additionally to quantify its concentration in the sample.
There are several known methods for the detection of products in quantitative nucleic acid amplification. According to one of these methods, in order to indirectly measure the amount of nucleic acid during RNR, the intensity of a fluorescence emission of the analyte is acquired and a growth curve is created indicative of the intensity of the fluorescence emission over the RNR cycle range. The increase of the growth curve signal above the initially stationary baseline is used to determine a quantification cycle Cq in each reaction.
The determination of the quantification cycle Cq based on the growth curve using various CT methods is the subject of multiple patent applications:

EP 2719770 A1 discloses a method of detecting a presence and/or measuring a quantity of an analyte in a sample by PCR as well as a respective analyzer. In particular, signal normalization of the growth curve taking into account a maximum growth value of the signal is disclosed.

US7788039B2 discloses a method for determining an amount of target nucleic acid in a sample. An initial amount of the target is calculated according to a calibration equation using an initial amount of the standard, the target and standard growth curve values, where the calibration equation is a non-linear equation.

EP1798652B1 discloses a mathematical algorithm for creating a growth signal from measurement data.

EP1798542A1 shows a method for determining the presence of a nucleic acid in a sample wherein a digital value is selected on the growth signal and the presence of the nucleic acid is determined from a comparison of the selected digital value with calibrated digital values.

"Analyzing real-time PCR data by the comparative CT method", Thomas D Schmittgen et al., Nature Protocols 3, - 1101 - 1108 (2008) provides an overview of the comparative $C_T$ method for quantitative gene expression studies.
nucleic acid amplification systems are commercially available from various vendors based on various nucleic acid amplification methods and employed for medical applications such as the detection of infectious diseases. Therefore, reliability and precision of nucleic acid amplification methods is of utmost importance.
Embodiments of the invention therefore aim to provide an improved analysis method using nucleic acid amplification and a respective system that enable improved robustness, precision, accuracy, linearity and qualitative discrimination of a nucleic acid amplification analysis.

**SUMMARY OF THE INVENTION**

The invention as defined by the appended claims concerns an analysis method for a real-time polymerase chain reaction is provided for detecting a presence and/or measuring a quantity of an analyte in a sample as claimed in claim 1. Further, a system for analyzing a nucleic acid amplification reaction of an analyte is provided.

Embodiments of the invention relate to an analysis method for a real-time nucleic acid amplification RNR using a combined threshold function CTF over the RNR cycle range, the CTF comprising at least two different threshold levels. The CTF is obtained by calculation on the basis of the at least two different threshold levels.

The calculation may be performed before or after the acquisition of the intensity of the fluorescence emission and the creation of the growth curve. For example, the calculation is performed once and the resultant CTF is stored in an electronic memory for reading out the CTF when it is needed for determining the quantification cycle number Cq of the RNR. Alternatively the CTF is calculated after the acquisition of the intensity of the fluorescent emission and/or after the creation of the growth curve before the determination of the quantification cycle number.

Embodiments of the invention are particularly advantageous as combining at least two different threshold levels into the CTF enables improvement of the precision of the RNR over a wide range of analyte concentrations and/or limit the effect of the presence of interfering substances.

Moreover, embodiments of the present invention are particularly advantageous as the occurrence of false negatives for weak analyte concentrations and erroneous Cq values for high analyte concentrations are prevented thus increasing the reliable working range of the RNR analysis.

In accordance with embodiments of the invention at least one of the CT threshold levels is a constant value over the RNR cycle range. The constant value may be given by a relative intercept increase (RII-method), a partial growth threshold (PGT-method), a multiple of standard deviation of the growth curve above the baseline, a manually set threshold or a constant function.

In accordance with embodiments of the invention the CTF is obtained from the at least two different threshold levels by combining the threshold levels over the RNR cycle range, such as by superposition, linear or non-linear combination. Alternatively or in addition the CTF is obtained by dividing the RNR cycle range into RNR cycle intervals and calculating a separate CTF per interval using at least two different threshold levels per interval wherein the at least two different threshold levels can vary from interval to interval.

In accordance with an embodiment of the invention the CTF is a combination, such as a linear combination, of two threshold levels over the entire RNR cycle range. One of the threshold levels delivers a dominant contribution to the CTF at early reaction cycles of the RNR, such as for cycle numbers below 10, 15 or 20, whereas one of the threshold levels delivers a dominant contribution to the CTF at late reaction cycles of the RNR, such as for cycle numbers greater than 30, 40 or 50. In accordance with embodiments of the invention the CTF transitions from an initial threshold level to a final threshold level over the RNR cycle range. In other words, the contribution of the initial threshold level to the CTF may be dominant for early reaction cycles of the RNR such as for cycle number 0 or 1 and the contribution of the final threshold level to the CTF may be substantially below the contribution of the initial threshold level such as 0 at early reaction cycles of the RNR, such as for cycle number 0 or 1. Analogously, the contribution of the final threshold level at late reaction cycles of the RNR to the CTF may be substantially above the contribution of the initial threshold level. The transition of the CTF from the initial threshold level to the final threshold level over the RNR cycle range may be a step or multi-step transition, a linear transition, a polynomial transition, an exponential transition or a combination thereof.

In accordance with embodiments of the invention the initial threshold level itself comprises a first combination A of at least two different threshold levels. Alternatively or in addition, the final threshold level itself comprises a second combination B of at least two different threshold levels where the threshold levels that are combined into the initial threshold level may be the same or different threshold levels than the threshold levels that are combined into the final threshold level.

In accordance with an embodiment of the invention one of the initial and final threshold levels is not a combination of threshold levels but a single threshold level.

In accordance with embodiments of the invention the initial threshold level comprises at least one threshold level that is adapted for precise determination of the quantification cycle number for a strong growth curve, i.e. a growth curve obtained for a high analyte concentration and/or low concentration of an interference substance.

On the other hand, the final threshold level comprises at least one threshold level that is adapted for precise determination of the quantification cycle number for late increases, i.e. a low analyte concentration and/or high concentration of an interference substance. Due to the transition of the CTF from the initial threshold level to the final threshold level over the RNR cycle range a precise determination of the quantification cycle number is enabled both for early and late signal increases.

In accordance with embodiments of the invention the initial threshold level has a starting point with a y-coordinate below a y-coordinate of an end point of the final threshold level resulting in an increasing combined threshold function with a positive slope or the initial threshold level has a starting point with a y-coordinate above a y-coordinate of an end point of the final threshold level resulting in a decreasing combined threshold function, i.e. in a combined threshold function that has a negative slope over the RNR cycle range.

In accordance with embodiments of the invention the y-coordinate of the starting point and the y-coordinate of the end point are used to calculate the combined threshold function by a linear, polynomial, exponential, logarithmic or other transition between the two y-coordinates. For example, the two y-coordinates are stored in an electronic memory and read from the electronic memory for calculating the combined threshold function by means of linear interpolation between the two y-coordinates.

In accordance with embodiments of the invention the CTF is a constant over the RNR cycle range. For example each of the threshold levels provides a constant and the combined threshold function is calculated as a linear combination of the two constants such as an average or a weighted average of the two constants. For example, one of the threshold levels is adapted for precise determination of the quantification cycle number for an early signal increase whereas the other of the two threshold levels may be adapted for precise determination of the quantification cycle number for a late signal increase hence for CT determination at late reaction cycles of the RNR.

In accordance with another aspect of the invention a system for analyzing a nucleic acid amplification reaction of an analyte is provided, the system comprising: a detection system configured to acquire the intensity of fluorescence emission of the analyte for each amplification reaction cycle of the RNR and a processing unit configured to: create a growth curve indicative of the intensity of the fluorescent emission over a range of reaction cycles of the RNR, provide a combined threshold function CTF comprising at least two different threshold levels; and determine a quantification cycle number Cq of the RNR, the quantification cycle number Cq being indicative of a quantitative and/or qualitative analysis result of the growth curve as the cycle number corresponding to an intersection of the growth curve with the combined threshold function.

In accordance with embodiments of the invention the processing unit provides the combined threshold function by reading data from an electronic memory of the system that is descriptive of the combined threshold function and calculating the combined threshold function using this data and/or the growth curve. For example, the data stored in the electronic memory indicates the y-coordinate of the starting point of the initial threshold level and the end point of the final threshold level and the calculation of the combined threshold function by the processor is performed by means of an interpolation between the y-coordinates over the RNR cycle range.

Embodiments of the invention may be particularly advantageous as the accuracy and precision of the analysis may be increased over a wide concentration range of the analyte and/or robustness against interferences.

## BRIEF DESCRIPTION OF THE DRAWINGS

In the following embodiments of the invention will be described in greater detail, by way of example only, making reference to the drawings in which:

Fig. 1      is a block diagram illustrating a schematic of a RNR analyzer for analyzing a sample, such as a biological sample,

Fig. 2      illustrates schematically a RNR growth curve of an analyte for each RNR cycle,

Fig. 3A      illustrates schematically two different RNR growth curves, two threshold levels and a resultant CTF,

Fig. 3B      illustrates schematically two different RNR growth curves, two threshold levels and a resultant CTF,

Fig. 4      illustrates an alternative CTF for the growth curves of Fig. 3,

Fig. 5      shows a window that is displayed by an analyzer with a negatively sloped CTF,

Fig. 6      shows a window that is displayed by an analyzer with a negatively sloped CTF, the CTF transitioning from RII as initial threshold level to PGT as final threshold level.

Fig. 7      shows a window that is displayed by an analyzer with a positively sloped CTF,

Fig. 8      shows examples for linear, quadratic and exponential transition between the initial and final threshold levels,

Fig. 9      shows various growth curves of a dilution series,

Fig. 10      shows a comparison of various $C_q$ values obtained using known threshold levels and the inventive CTF,

Fig. 11      shows various calibration curves of $C_q$ values as a function of concentration for known threshold levels and the inventive CTF,

Fig. 12      shows growth curves of interferences,

Fig. 13      shows thresholds of interferences, and

Fig. 14      shows $C_q$ values as function of interference growth curve

## DETAILED DESCRIPTION

Throughout the following detailed description of embodiments of the invention identical or like elements are designated by identical reference numerals.

The term 'baseline' refers to the initial portion of the growth curve which shows no signal increase.

The term 'intercept' or 'intercept value' as understood herein is the signal offset value of the growth curve at an early cycle number, i.e. 0 that is obtained by extrapolation.

The term 'saturation line' refers to the portion of the growth curve in the plateau region after the exponential growth phase which is also referred to as exponential amplification phase and after the leveling off stage.

The term 'threshold level' as used herein encompasses a threshold level for the growth curve.

A 'combined threshold function' or 'CTF' as understood herein encompasses a mathematical function over the cycle range that is composed of at least two different threshold levels. According to one embodiment, a CTF may be a linear or logarithmic combination of two different threshold levels.

In one embodiment, the Y-value, i.e. the threshold level, returned by the CTF for a given X-value, i.e. cycle number, may transition from one of the two different threshold levels to the other one.

According to one embodiment, a CTF may transition from a fist combination (e.g. linear or logarithmic) of at least two different threshold levels to a second combination (e.g. linear or logarithmic) of at least two different threshold levels.

The term 'real-time nucleic acid amplification RNR cycle range' as understood herein refers to the range of cycle numbers that is executed for acquiring the intensity values for the creation of the growth curve. For example, the RNR cycle range may be a predefined fixed number, such as 30, 40 or 50. Setting the RNR cycle range is a tradeoff between throughput of the analysis system for analyzing the nucleic acid amplification reaction and the sensitivity of the analysis system for the determination of a quantification cycle number for weak signals.

In accordance with an embodiment of the invention a threshold method is implemented as a relative intercept increase RII. For example, the RII is a parallel above the baseline.

In accordance with embodiments of the invention the RII may have the following format:

$$y(x) = B + r \cdot I \qquad (1)$$

B: baseline function, typically linear
I: intercept extrapolation to cycle number 0
r: positive percentage parameter, e.g. 50%

In accordance with embodiments of the invention a threshold method may be implemented as a partial growth threshold PGT that depends on the signal difference of the growth curve between the saturation line and the baseline, i.e. the growth from the baseline to the saturation line. The PGT may return a constant Y-value over the entire range of cycle numbers.

In accordance with embodiments of the invention the PGT may have the following form:

$$y(x) = B + p \cdot G = B + p \cdot (S - B) \qquad (2)$$

B: baseline function
S: Saturation line
G: growth from baseline to saturation line
p: parameter preferably between 0% and 100%, e.g. 5%

In accordance with an embodiment of the invention a threshold method is given by the standard deviation of the growth curve above the baseline and may have the following form:

$$y(x) = B + f \cdot D \qquad (3)$$

B: baseline function
f: Multiplying factor parameter, e.g. 10
D: Standard deviation of growth curve

In accordance with embodiments of the invention a threshold method is given by a threshold value that is pre-set or manually entered by a user.

In accordance with an embodiment of the invention the threshold method is a constant value that is constant over the RNR cycle range, for example:

$$y(x) = C \qquad (4)$$

C:     a predefined constant based on empirical data.

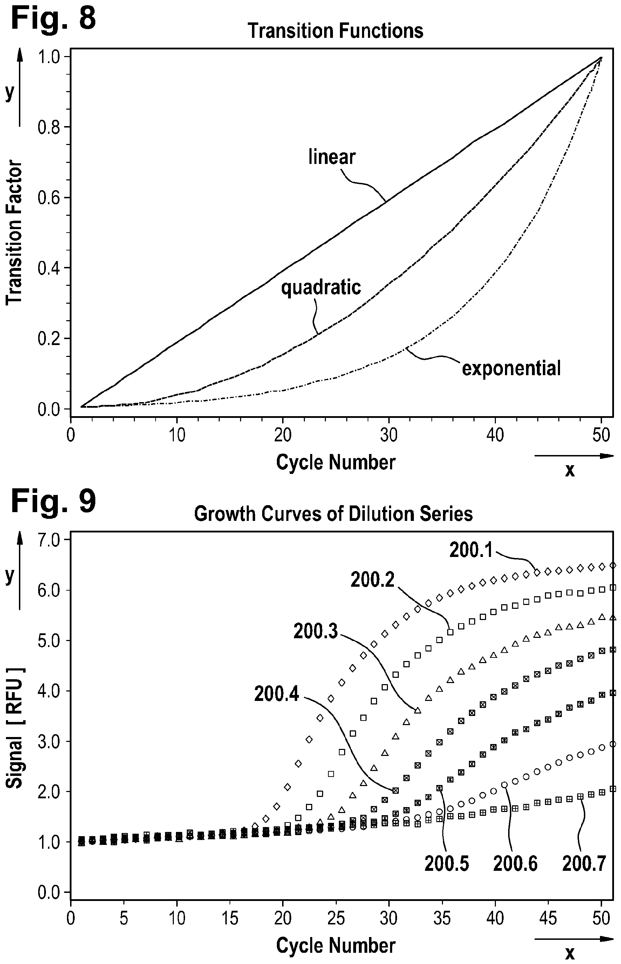

In accordance with embodiments of the invention the transition from one of the at least two different threshold levels, i.e. the initial threshold level, to the other one of the at least two different threshold levels, i.e. the final threshold level, is such by a step or multi-step, linear, polynomial and/or exponential transition as illustrated on figure 8.

For example, a CTF with a linear transition from the initial threshold level $y_1$ to the final threshold level $y_2$ may have the following form:

$$CTF(x) = y_1 + (y_2 - y_1) \cdot \frac{x - x_1}{x_2 - x_1} \qquad (5)$$

where:

$x_1$:     early reaction cycle number, such as 0 or 1;
$x_2$:     late reaction cycle number, such as 30, 40 or 50;
$y_1$:     initial threshold level;
$y_2$:     final threshold level.

In accordance with an embodiment of the invention the CTF may have the following format for implementation of a polynomial transition:

$$CTF(x) = y_1 + (y_2 - y_1) \cdot \left(\frac{x - x_1}{x_2 - x_1}\right)^p \qquad (6)$$

where:

p:     Fixed polynomial order, such as 2 for a quadratic polynomial function.

In accordance with an embodiment of the invention the CTF may have the following format for implementation of an exponential transition:

$$CTF(x) = e^{\frac{x_2 \cdot ln(y_1) - x_1 \cdot ln(y_2)}{x_2 - x_1}} \cdot \left(\frac{y_2}{y_1}\right)^{\frac{x}{x_2 - x_1}} \qquad (7)$$

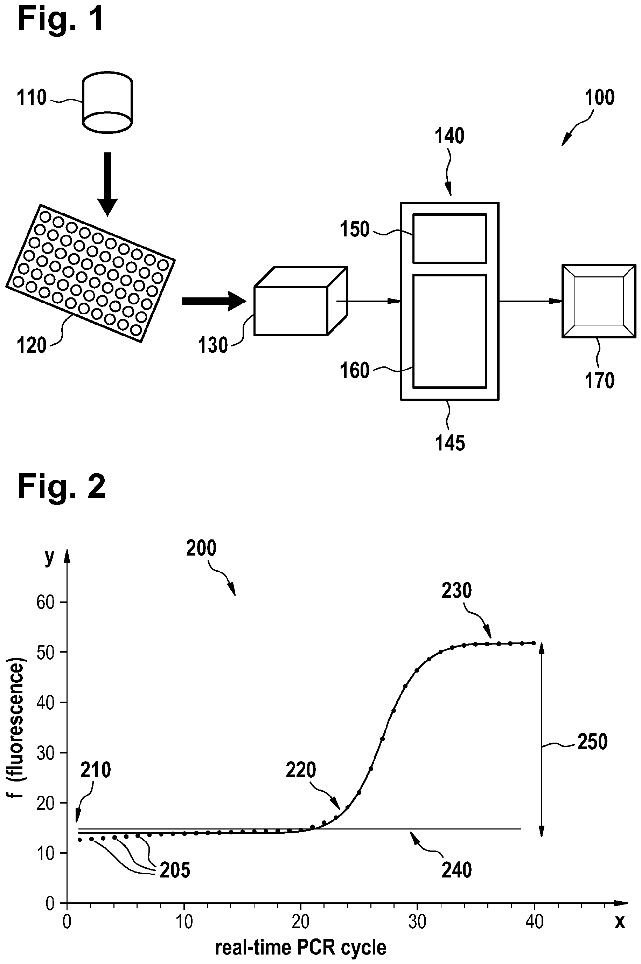

Figure 1 shows schematics of the RNR system 100 for amplification and simultaneous quantification of an analyte in real time. Using RNR method, a signal is created and detected during the amplification process. The signal generally represents the amount of any analyte created during amplification and thus present in the sample. The sample is a liquid that may contain the analyte and/or other products of the amplification reaction. The RNR system comprise a thermal cycler block 120, an excitation light source 110, a detector system 130 for collecting the RNR signal in real time, and a data processing system 140 comprising a processing unit and a memory 150 for storing the RNR signal and program instructions 160 for analyzing the RNR signal, and a unit 170 for displaying the signal and outputting a result of the analysis.

The analyte is conjugated to a fluorescent dye and the sample is loaded into the thermal cycler block 120. A thermal cycler block 120 can be a conventional design sample block, which comprise 96 wells and is able to hold up to 96 samples. The sample is illuminated with the fluorescence excitation source 110, and the raw fluorescence data is measured by the RNR detector system 130 for each RNR cycle number. The RNR detector 130 is suitable to collect the RNR fluorescence signal emitted by one or more fluorescent dyes. The measured data is collected in data processing system memory unit 150, and can be displayed on the display unit 170 as an un-normalized RNR growth curve, or alternatively as a normalized RNR growth curve.

Figure 2 shows the schematic example of a growth curve 200 representing the RNR signal taken for each RNR cycle. The diagram includes a Cartesian coordinate system where the abscissa is designated as the x-axis and the ordinate is designated as the y-axis and where x is the cycle number of the RNR and y is the intensity of the fluorescent emission.

Figure 2 also shows fluorescence intensity values 205 as a dotted line, where each of the intensity values 205 has been acquired by performing a fluorescence emission intensity measurement of the ongoing amplification reaction.

The intensity values 205 are modeled using a mathematical growth curve model formula or another kind of interpolation and/or interpolation. The RNR signal is showing as growth curve 200 in figure 2. Here the intercept value 210 is the RNR signal offset value when the RNR cycle number is zero.

The baseline 240 is the RNR signal during the initial cycles of the RNR reaction, typically measured between cycles 1 and 15, where there is no detectable increase in fluorescence due to RNR reaction products. The baseline 240 is the baseline function B (cf. equations 1 to 3). The pre-defined threshold 220 is used to determine a cycle number at which the RNR signal exceeds the baseline 240 of the RNR reaction, i.e. the cycle in which there is the first detectable significant increase in fluorescence, which is about $c_q = 22$ here. The threshold 220 is given by CTF (cf. equations 5 to 7).

The maximum growth value 250 is a difference between a maximum intensity of the RNR signal at a plateau region 230 and the RNR signal at the baseline 240. The plateau stage 230 is the RNR signal during final cycles of the RNR reaction.

The intensity of the RNR signal at a plateau region 230 is S and the maximum growth value 250 is the growth G in the above equation 2.

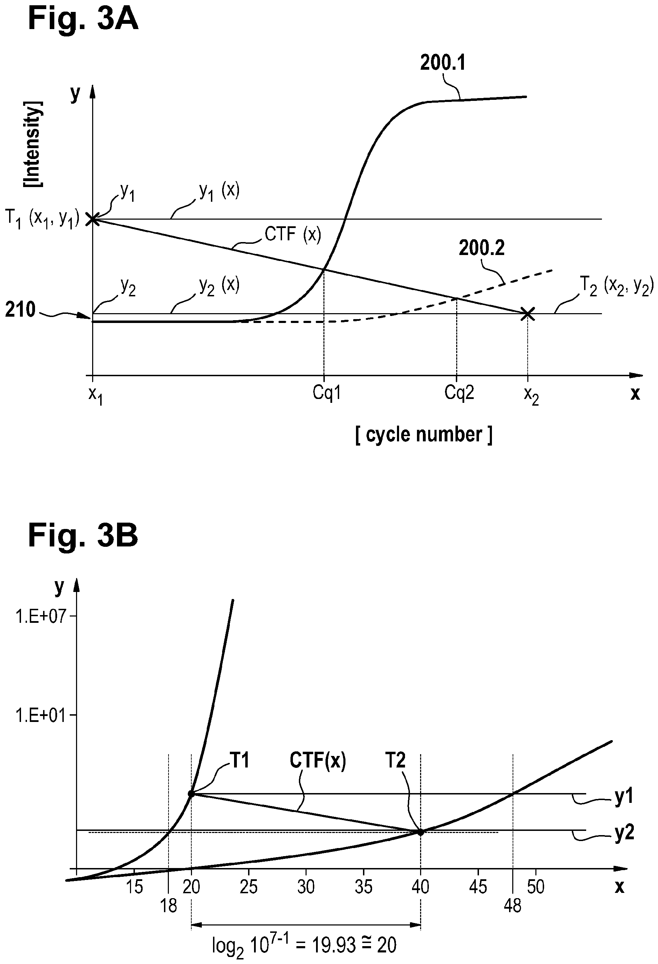

Fig. 3A shows a diagram with a graphical representation of a growth curve 200.1 and another growth curve 200.2 which is shown as a dashed line. The diagram includes a Cartesian coordinate system where the abscissa is designated as the x-axis and the ordinate is designated as the y-axis analogous to the diagram shown in Fig. 2 where x is the cycle number of the RNR and y is the intensity of the fluorescent emission.

The diagram shows a first threshold level y1 and a second threshold level y2.

The threshold y1 may be defined by entering a starting point $T_1(x_1, y_1)$; likewise the threshold level y2 may be defined by entering an end point $T_2 (x_2, y_2)$.

In the embodiment considered here the y-coordinate of the starting point $T_1$ that determines the threshold level y1 is greater than the respective coordinate of $y_2$ of the end point $T_2$ such that y1 is above the other threshold level y2 as illustrated in Fig. 3A.

The growth curve 200.1 originates from a sample concentration at the upper end of the dynamic range of the RNR whereas the sample concentration for the growth curve 200.2 is at the lower end of the dynamic range. As a consequence, the exponential phase of the growth curve 200.1 occurs in a much lower cycle number range that is the case for the growth curve 200.2 as illustrated in Fig. 3A.

Application of the threshold level y1 alone for the determination of $c_q$ of growth curves 200.1 and 200.2 would result in the detection of $c_q$ only for growth curve 200.1 but would fail to detect a $c_q$ value (or lead to very late detection) for growth curve 200.2 as the latter is too weak to reach the intensity $y_1$ in its exponential phase and/or plateau region within the RNR cycle range.

On the other hand, usage of threshold level y2 alone would lead to the detection of a $c_q$ value for growth curve 200.2 but would be unreliable as regards detection for a $c_q$ value for growth curve 200.1 as $y_2$ is close to the baseline such that variations could lead to an imprecise $C_q$ detection for the growth curve 200.1.

This situation is remedied by combining threshold levels y1 and y2 which provides a combined threshold function CTF(x). In the example considered here, the CTF is in accordance with above equation 5 such that CTF has a linear transition from $y_1$ to $y_2$ over the x-coordinate range $x_1$ to $x_2$ as illustrated in Fig. 3A. This has the beneficial effect that the CTF intersects with both growth curves 200.1 and 200.2 within their respective exponential growth phases resulting in a reliable and precise detection of the respective quantification cycle numbers $c_{q1}$ and $c_{q2}$.

Hence, the combined threshold function CTF(x) is calculated by combining the threshold levels y1 and y2. In the embodiment considered here this is a linear combination providing a linear transition from $T_1$ to $T_2$ along the x-axis. The RNR cycle range can be set to be equal to the x-coordinate range $x_1$ to $x_2$ in the embodiment considered here as additional RNR cycles above cycle $x_2$ would not provide a significant contribution to the quantification cycle number determination but merely reduce system throughput.

The calculation of the CTF can be performed by the RNR system 100 (cf. Fig. 1). For example, the coordinates of the points $T_1$ to $T_2$ are stored in the memory 150. By execution of the program instructions 160 $T_1$ to $T_2$ are read from the memory 150 and the combined threshold function CTF is calculated such as in accordance with equation 5, 6 or 7. Alternatively the combined threshold function CTF is stored in the memory 150 by means of data that is descriptive the CTF, such as in tabular form.

In the example illustrated in Fig. 3A the CTF transitions from the initial threshold level y1 to the final threshold level y2. In the example considered here the transition is linear and results in a negatively sloped CTF whereby the initial threshold level y1 provides the initial starting point $T_1$ at cycle number $x_1 = 0$ and the final threshold level y2 provides the end point $T_2$ of the CTF at cycle number $x_2$.

Fig. 3B is illustrative of an alternative choice of the levels y1 and y2. In the example considered here usage of threshold level y1 alone would not result in a false negative as it would be the case for the Fig. 3 example. However, the point of intersection of the growth curve 200.2 with the CTF threshold level y1 is only at a late cycle number $C_{q2}$, i.e. at x = 48 instead of x = 40 (i.e. ca. $\log_2 10^6 = 19.93 \cong 20$ cycles later than $C_{q1}$ corresponding to a ratio of $10^6$ between the concentration of the first respectively second analyte). This is disadvantageous as a relatively large number of cycles have to be executed for a qualitative result reducing throughput of the analysis system. Another disadvantage is that the point of intersection between CT threshold level y1 and the growth curve 200.2 may occur after the exponential growth phase of the growth curve 200.2 where the RNR process is in its leveling off stage (i.e. the amount of nucleic acid is no longer doubled at every cycle) leading to false $C_q$ values. This situation is improved by using the CTF that results from the combination of y1 and y2 as the point of intersection and hence $C_q$ for growth curve 200.2 is moved to x = 40.

Alternatively CTF(x) can be in accordance with above equation 6 or 7 or it can be another monotonous or step-function.

In accordance with embodiments of the invention the threshold level y1 is in accordance with equation 2 thus taking into account the signal level S of the saturation line in the plateau phase whereas the threshold level y2 is in accordance with equation 1 taking into account the intercept value I rather than S.

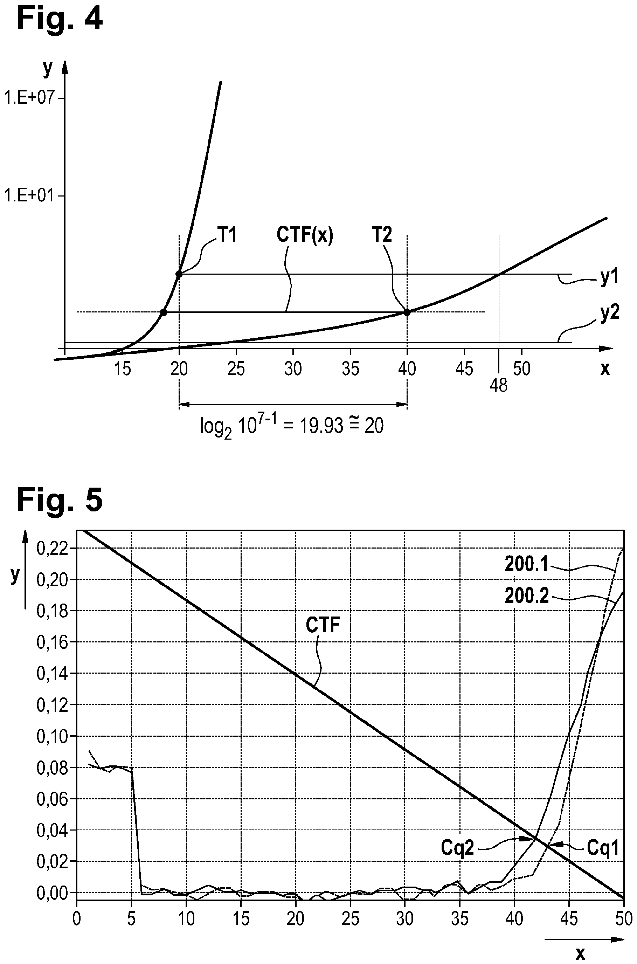

Fig. 4 illustrates an alternative embodiment for the combination of the thresholds $y_1$ and $y_2$ by means of a linear combination, such as

CTF (x) = 0.5 y1 + 0.5 y2 which is a constant and thus there is no transition of CTF in this case, wherein the threshold level y1 is adequate for early reaction cycles of the RNR while the threshold level y2 being adequate for late reaction cycles of the RNR, the combination of which resulting in a CTF adequate over a wide cycle range. For example, an adequate choice of y1 and y2 is to choose y1 and y2 such that y1 > y2 as illustrated in Fig. 4.

The resultant CTF is illustrated in Fig. 4 as well as the quantitation cycle numbers $C_q$ which are thus detected for the growth curves 200.1 and 200. 2.

Fig. 5 is illustrative of the window which is output on the display 170 (cf. Fig. 1) with a CTF that has a negative slope for the detection of $c_{q1}$ and $c_{q2}$ similar to the embodiment of Fig. 3.

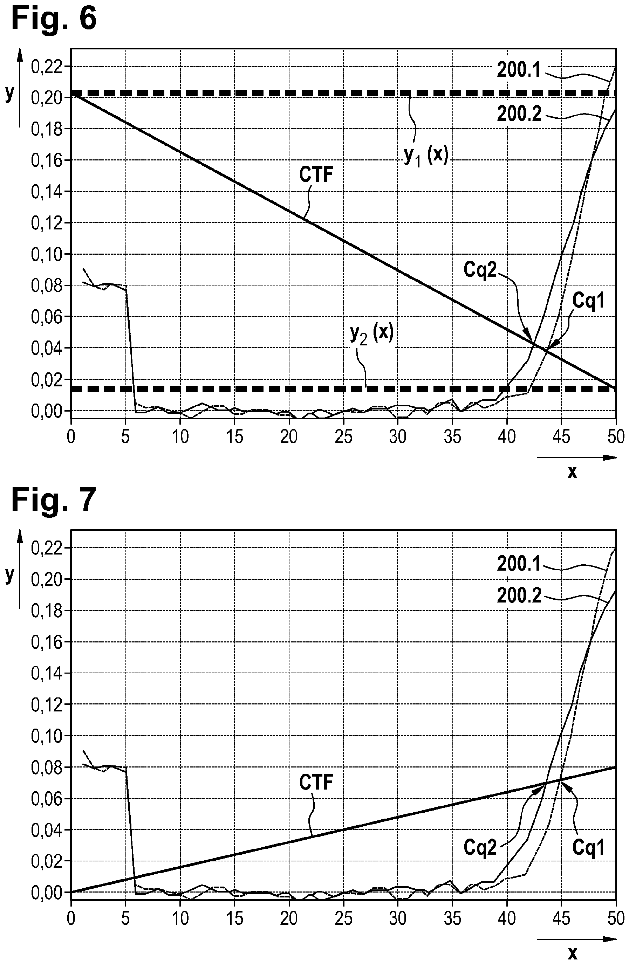

Fig. 6 illustrates a further embodiment similar to the embodiments of Fig. 3A and Fig. 3B, where the initial threshold level y1 - being the RII in accordance with above equation 1 - linearly transitioning in accordance with above equation 5 to the final threshold level y2 - being the PGT in accordance with above equation 2.

Fig. 7 is illustrative of an embodiment where the CTF has a positive slope.

Fig. 8 illustrates the transition of the contributions of thresholds y1 and y2 to CTF for a linear, quadratic (p = 2) and exponential transition in accordance with equations 5, 6 and 7, respectively.

Fig. 9 shows growth curves 200.1 to 200.7 for a dilution series where the growth curve 200.1 is obtained for the highest concentration of the analyte and the consecutive growth curves 200.2, 200.3 etc are obtained for decreasing concentrations of the analyte where the analyte concentration is decreased by one order of magnitude from one growth curve to the next, which illustrates the dynamic range of the analyzer (cf. RNR system 100 of Fig. 1).

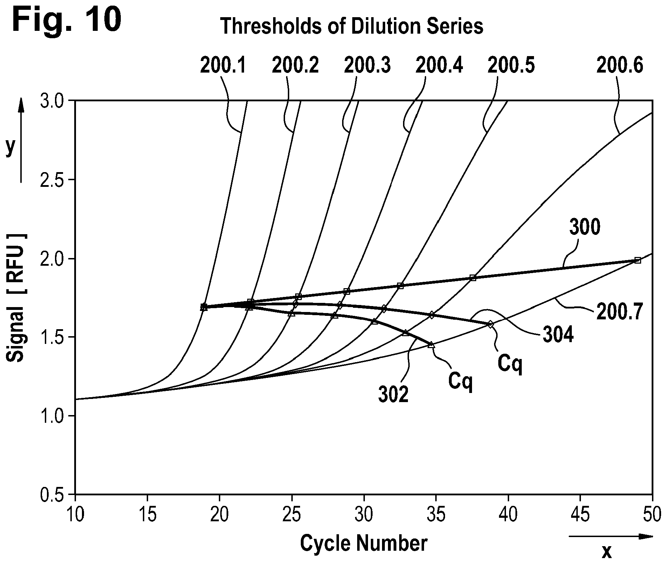

Fig. 10 is illustrative of the $c_q$-values obtained from growth curves 200.1 to 200.7 for three cases:

    i. the curve 300 shows the $c_q$ values that are obtained when an RII alone is used with r = 50% in accordance with equation 1.

    ii. the curve 302 shows the $c_q$ values that are obtained if a PGT alone is used with p = 0.1 in accordance with equation 2.

    iii. Curve 304 is obtained when a CTF is used that combines these RII and PGT, e.g. in accordance with equation 5, 6 or 7, resulting in approximately equidistant $c_q$ values for the dilution series.

As it can be observed in figure 10, the use of a standard CT method with a fixed threshold height, e.g RII 0.5 leads to a relative $C_q$ delay for low concentration curves because of the less steep increase. On the other hand the use of a growth related CT method like PGT might over-compensate the effect of less prominent growth by early detection of the $C_q$ values.

The equidistant $C_q$ values that are obtained in the above case iii. reflect the constant relative concentration of the respective analytes corresponding to consequent growth curves 200.1 to 200.7 (i.e. one order of magnitude for consecutive curves) which is due to the fact that the point of intersection of the CTF with the respective growth curves is always within the exponential growth phase irrespective of the degree of dilution of the analyte over an extremely broad concentration range. This in turn implies that the precision of the $C_q$ determination is substantially increased in comparison to above cases i. and ii. due to the fact that the growth curve has the lowest amount of noise within the exponential growth phase and thus yields most accurate results.

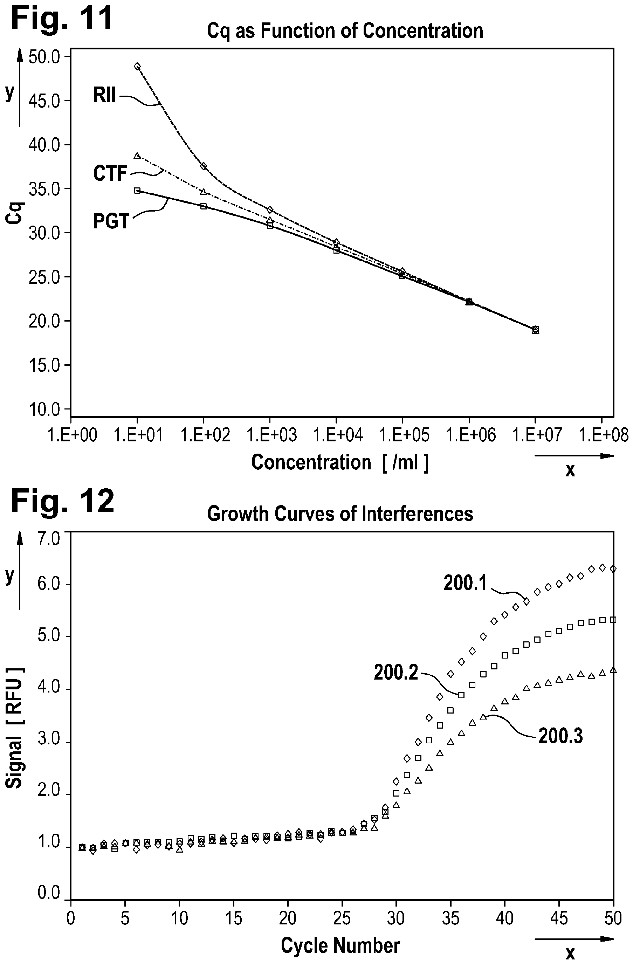

Fig. 11 shows calibration curves of the data of Fig. 10 if the RII or the PGT is used alone as well as for the combined CTF curve that provides an almost perfect linear calibration curve as apparent from Fig. 11. If a single threshold method such as RII or PGT alone is used there is a significant deviation of the calibration curve from the theoretic linear law that relates the $C_q$ value to the initial analyte concentration of the sample on a logarithmic scale as apparent from Fig. 11.

Fig. 12 shows three growth curves 200.1, 200.2 and 200.3 for the same concentration of the analyte but different concentrations of an interference substance that affects the RNR reaction.

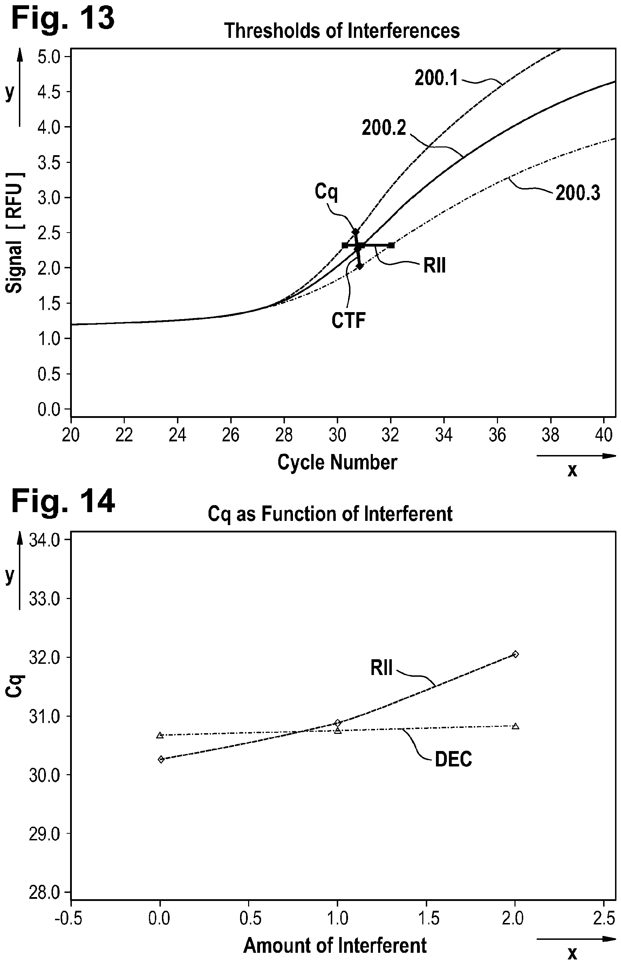

Fig. 13 shows the resultant $c_q$ values that are obtained for the growth curves 200.1 to 200.3 of Fig. 12 if a flat threshold method RII is used and when a CTF with a negative slope is used such as in accordance with equations 5, 6 or 7.

As apparent from Fig. 13, the $C_q$ values obtained for the three growth curves 200.1, 200.2 and 200.3 vary between 30 and 32 if the threshold method RII alone is used resulting in a respective large error. In contrast, as also illustrated in Fig. 13, the $C_q$ values obtained for these curves using the negatively sloped CTF results in almost identical $C_q$ values for all three curves thus greatly improving the precision even if various concentrations of interference substances are present in the sample. This is also illustrated in Fig. 14 which shows the $c_q$ values of identical sample concentrations as a function of interferent concentration.

**Claims**

1.  An analysis method for a real-time nucleic acid amplification reaction RNR, the method comprising:

    - acquiring an intensity of a fluorescence emission of an analyte for each amplification reaction cycle of the RNR;
    - creating a growth curve (200) indicative of the intensity of the fluorescence emission over a RNR cycle range,
    - calculating a combined threshold function (CTF) over the RNR cycle range comprising at least two different threshold levels, wherein the combined threshold function (CTF) is dependent on the cycle number of the RNR, the combined threshold function (CTF) transitioning from an initial threshold level (y1) to a final threshold level (y2), the initial threshold level (y1) being different than the final threshold level (y2),

      the initial threshold level (y1) being B + r x I, wherein B is a baseline function, the baseline being the initial portion of the growth curve which shows no signal increase, r is a positive percentage parameter, and I is an intercept extrapolation to cycle number 0,
      the final threshold level (y2) being $y(x) = B + p \cdot G = B + p \cdot (S - B)$, wherein B is the baseline function, S is the saturation line of the growth curve, G is the growth from the baseline to the saturation line, and p is a parameter between 0% and 100%, wherein the initial threshold level (y1) is above the final threshold level (y2),
      wherein the combined threshold function is selected from a group of three functions comprising:

      i. a CTF with a linear transition from the initial threshold level ($y_1$) to the final threshold level ($y_2$) according to:
      $$CTF(x) = y_1 + (y_2 - y_1) \cdot \frac{x - x_1}{x_2 - x_1}$$
      , where x1 is an early reaction cycle number equal to 0 or 1, x2 is a late reaction cycle number larger than 30, y1 is the initial threshold level, y2 is the final threshold level.

      ii. a CTF with a polynomial transition from the initial threshold level ($y_1$) to the final threshold level ($y_2$) according to: $CTF(x) = y_1 + (y_2 - y_1) \cdot \left(\frac{x - x_1}{x_2 - x_1}\right)^p$ , where p is a fixed polynomial order, wherein p is 2 for a quadratic polynomial function;

      iii. a CTF with an exponential transition from the initial threshold level ($y_1$) to the final threshold level ($y_2$) according to
      $$CTF(x) = e^{\frac{x_2 \cdot ln(y_1) - x_1 \cdot ln(y_2)}{x_2 - x_1}} \cdot \left(\frac{y_2}{y_1}\right)^{\frac{x}{x_2 - x_1}}$$
      ;

    - determining a quantification cycle number (Cq) of the RNR, the quantification cycle number (Cq) being indicative of a quantitative and/or qualitative analysis result of the growth curve (200) as the cycle number corresponding to an intersection of the growth curve (200) with the combined threshold function (CTF).

2.  The analysis method according to claim 1, wherein:

    - the initial threshold level (y1) comprises at least one method adequate for early reaction cycles of the RNR; and/or
    - the final threshold level (y2) comprises at least one method adequate for late reaction cycles of the RNR.

3. The analysis method according to any one of the preceding claims, wherein:

   - the initial threshold level (y1) has a starting point ($T_1$) with an y-coordinate above an y-coordinate of an end point ($T_2$) of the final threshold level (y2) resulting a decreasing combined threshold function (CTF).

4. The analysis method according to any one of the preceding claims, wherein the combination comprises at least one threshold level adequate for early reaction cycles of the RNR and at least one threshold level adequate for late reaction cycles of the RNR.

5. The analysis method according one of the preceding claims, wherein the real-time nucleic acid amplification reaction RNR is a real-time polymerase chain reaction PCR.

6. A system for analyzing a real-time nucleic acid amplification reaction RNR of an analyte, said system comprising:

   - a detection system (130) configured to acquire an intensity of a fluorescence emission of the analyte for each amplification reaction cycle of the RNR;
   - a processing unit (145) configured to:

     • create a growth curve (200) indicative of the intensity of the fluorescence emission over a range of reaction cycles of the RNR;

     - provide a combined threshold function (CTF) over the RNR cycle range comprising at least two different threshold levels, wherein the combined threshold function (CTF) is dependent on the cycle number of the RNR, the combined threshold function (CTF) transitioning from an initial threshold level (y1) to a final threshold level (y2), the initial threshold level (y1) being different than the final threshold level (y2),

       the **initial** threshold level (**y1**) being B + r x I, wherein B is a baseline function, the baseline being the initial portion of the growth curve which shows no signal increase, r is a positive percentage parameter, and I is an intercept extrapolation to cycle number 0,
       the **final** threshold level (**y2**) being $y(x) = B + p \cdot G = B + p \cdot (S - B)$, wherein B is the baseline function, S is the saturation line of the growth curve, G is the growth from the baseline to the saturation line, and p is a parameter between 0% and 100%,
       wherein the initial threshold level (y1) is above the final threshold level (y2),

     - wherein the combined threshold function is selected from a group of three functions comprising:

       i. a CTF with a linear transition from the initial threshold level ($y_1$) to the final threshold level ($y_2$)

       $$CTF(x) = y_1 + (y_2 - y_1) \cdot \frac{x - x_1}{x_2 - x_1}$$

       according to: , where x1 is an early reaction cycle number equal to 0 or 1, x2 is a late reaction cycle number larger than 30, y1 is the initial threshold level, y2 is the final threshold level.
       ii. a CTF with a polynomial transition from the initial threshold level ($y_1$) to the final threshold level

       ($y_2$) according to: $CTF(x) = y_1 + (y_2 - y_1) \cdot \left(\frac{x - x_1}{x_2 - x_1}\right)^p$ , where p is a fixed polynomial order, wherein p is 2 for a quadratic polynomial function;
       iii. a CTF with an exponential transition from the initial threshold level ($y_1$) to the final threshold

       level ($y_2$) according to $CTF(x) = e^{\frac{x_2 \cdot ln(y_1) - x_1 \cdot ln(y_2)}{x_2 - x_1}} \cdot \left(\frac{y_2}{y_1}\right)^{\frac{x}{x_2 - x_1}}$ ; and

     • determine a quantification cycle number (Cq) of the RNR, the quantification cycle number (Cq) being indicative of a quantitative and/or qualitative analysis result of the growth curve (200) as the cycle number corresponding to an intersection of the growth curve (200) with the combined threshold function (CTF).

7. The system of claim 6, wherein the combined threshold function (CTF) is provided by the processing unit by reading data and/or instruction(s) from an electronic memory of the system that is descriptive of the combined threshold function (CTF) and/or by calculating the combined threshold function (CTF).

8. The system according to claim 6 or 7, wherein the real-time nucleic acid amplification reaction RNR is a real-time polymerase chain reaction PCR.

9. An analysis method for a real-time nucleic acid amplification reaction RNR, the method comprising:

- acquiring an intensity of a fluorescence emission of an analyte for each amplification reaction cycle of the RNR;
- creating a growth curve (200) indicative of the intensity of the fluorescence emission over a RNR cycle range,
- calculating a combined threshold function (CTF) over the RNR cycle range comprising at least two different threshold levels, wherein the combined threshold function (CTF) is dependent on the cycle number of the RNR, the combined threshold function (CTF) transitioning from an initial threshold level (y1) to a final threshold level (y2), the initial threshold level (y1) being different than the final threshold level (y2),

the initial threshold level (y1) being $B + r \times I$, wherein B is a baseline function, the baseline being the initial portion of the growth curve which shows no signal increase, r is a positive percentage parameter, and I is an intercept extrapolation to cycle number 0,
the final threshold level (y2) being $y(x) = B + \rho \cdot G = B + p \cdot (S - B)$, wherein B is the baseline function, S is the saturation line of the growth curve, G is the growth from the baseline to the saturation line, and p is a parameter between 0% and 100%,
wherein the initial threshold level (y1) is below the final threshold level (y2),
wherein the combined threshold function is selected from a group of three functions comprising:

i. a CTF with a linear transition from the initial threshold level ($y_1$) to the final threshold level ($y_2$) according

$$CTF(x) = y_1 + (y_2 - y_1) \cdot \frac{x - x_1}{x_2 - x_1}$$

to: , where x1 is an early reaction cycle number equal to 0 or 1, x2 is a late reaction cycle number larger than 30, y1 is the initial threshold level, y2 is the final threshold level.
ii. a CTF with a polynomial transition from the initial threshold level ($y_1$) to the final threshold level ($y_2$)

according to: $CTF(x) = y_1 + (y_2 - y_1) \cdot \left(\frac{x - x_1}{x_2 - x_1}\right)^p$ , where p is a fixed polynomial order, wherein p is 2 for a quadratic polynomial function;
iii. a CTF with an exponential transition from the initial threshold level ($y_1$) to the final threshold level

($y_2$) according to $CTF(x) = e^{\frac{x_2 \cdot ln(y_1) - x_1 \cdot ln(y_2)}{x_2 - x_1}} \cdot \left(\frac{y_2}{y_1}\right)^{\frac{x}{x_2 - x_1}}$ ;

- determining a quantification cycle number (Cq) of the RNR, the quantification cycle number (Cq) being indicative of a quantitative and/or qualitative analysis result of the growth curve (200) as the cycle number corresponding to an intersection of the growth curve (200) with the combined threshold function (CTF).

10. A system for analyzing a real-time nucleic acid amplification reaction RNR of an analyte, said system comprising:

- a detection system (130) configured to acquire an intensity of a fluorescence emission of the analyte for each amplification reaction cycle of the RNR;
- a processing unit (145) configured to:

• create a growth curve (200) indicative of the intensity of the fluorescence emission over a range of reaction cycles of the RNR;

- provide a combined threshold function (CTF) over the RNR cycle range comprising at least two different threshold levels, wherein the combined threshold function (CTF) is dependent on the cycle number of the RNR,

the combined threshold function (CTF) transitioning from an initial threshold level (y1) to a final threshold level (y2), the initial threshold level (y1) being different than the final threshold level (y2), the initial threshold level (y1) being B + r x I, wherein B is a baseline function, the baseline being the initial portion of the growth curve which shows no signal increase, r is a positive percentage parameter, and I is an intercept extrapolation to cycle number 0,

the final threshold level (y2) being $y(x) = B + p \cdot G = B + p \cdot (S - B)$, wherein B is the baseline function, S is the saturation line of the growth curve, G is the growth from the baseline to the saturation line, and p is a parameter between 0% and 100%,

wherein the initial threshold level (y1) is **below** the final threshold level (y2),

- wherein the combined threshold function is selected from a group of three functions comprising:

i. a CTF with a linear transition from the initial threshold level ($y_1$) to the final threshold level ($y_2$)

$$CTF(x) = y_1 + (y_2 - y_1) \cdot \frac{x - x_1}{x_2 - x_1}$$

according to: , where x1 is an early reaction cycle number equal to 0 or 1, x2 is a late reaction cycle number larger than 30, y1 is the initial threshold level, y2 is the final threshold level.

ii. a CTF with a polynomial transition from the initial threshold level ($y_1$) to the final threshold level

($y_2$) according to: $CTF(x) = y_1 + (y_2 - y_1) \cdot \left(\frac{x - x_1}{x_2 - x_1}\right)^p$ , where p is a fixed polynomial order, wherein p is 2 for a quadratic polynomial function;

iii. a CTF with an exponential transition from the initial threshold level ($y_1$) to the final threshold

level ($y_2$) according to $CTF(x) = e^{\frac{x_2 \cdot ln(y_1) - x_1 \cdot ln(y_2)}{x_2 - x_1}} \cdot \left(\frac{y_2}{y_1}\right)^{\frac{x}{x_2 - x_1}}$ ; and

• determine a quantification cycle number (Cq) of the RNR, the quantification cycle number (Cq) being indicative of a quantitative and/or qualitative analysis result of the growth curve (200) as the cycle number corresponding to an intersection of the growth curve (200) with the combined threshold function (CTF).

**Patentansprüche**

1. Analyseverfahren für eine Echtzeit-Nukleinsäureamplifikationsreaktion RNR, wobei das Verfahren umfasst:

- Ermitteln einer Intensität einer Fluoreszenzemission eines Analyten für jeden Amplifikationsreaktionszyklus der RNR;
- Erzeugen einer Wachstumskurve (200), welche die Intensität der Fluoreszenzemission über einem RNR-Zyklusbereich angibt,
- Berechnen einer kombinierten Schwellenwertfunktion (CTF) über dem RNR-Zyklusbereich, der mindestens zwei verschiedene Schwellenwerthöhen umfasst, wobei die kombinierte Schwellenwertfunktion (CTF) von der Zyklusnummer der RNR abhängt, wobei die kombinierte Schwellenwertfunktion (CTF) von einer Höhe eines Anfangsschwellenwerts (y1) auf eine Höhe eines Endschwellenwerts (y2) übergeht, wobei die Höhe des Anfangsschwellenwerts (y1) von der Höhe des Endschwellenwerts (y2) verschieden ist,

wobei die Höhe des Anfangsschwellenwerts (y1) B + r x I ist, wobei B eine Grundlinienfunktion ist, wobei die Grundlinie der Anfangsabschnitt der Wachstumskurve ist, der keinen Signalanstieg zeigt, r ein positiver Prozentparameter ist und I eine Achsenabschnittsextrapolation für die Zyklusnummer 0 ist, wobei die Höhe des Endschwellenwerts (y2) $y(x) = B + p \cdot G = B + p \cdot (S - B)$ ist, wobei B die Grundlinienfunktion ist, S die Sättigungslinie der Wachstumskurve ist, G das Wachstum von der Grundlinie zur Sättigungslinie ist und p ein Parameter zwischen 0 % und 100 % ist, wobei die Höhe des Anfangsschwellenwerts (y1) über der Höhe des Endschwellenwerts (y2) liegt, wobei die kombinierte Schwellenwertfunktion ausgewählt ist aus einer Gruppe von drei Funktionen, umfassend:

i. eine CTF mit einem linearen Übergang von der Höhe des Anfangsschwellenwerts ($y_1$) auf die Höhe

$$CTF(x) = y_1 + (y_2 - y_1) \cdot \frac{x - x_1}{x_2 - x_1}$$

des Endschwellenwerts ($y_2$) gemäß: , wobei x1 eine Nummer eines frühen Reaktionszyklus gleich 0 oder 1 ist, x2 eine Nummer eines späten Reaktionszyklus größer 30 ist, $y_1$ die Höhe des Anfangsschwellenwerts ist, y2 die Höhe des Endschwellenwerts ist.

ii. eine CTF mit einem polynomischen Übergang von der Höhe des Anfangsschwellenwerts ($y_1$) auf

$$CTF(x) = y_1 + (y_2 - y_1) \cdot \left(\frac{x - x_1}{x_2 - x_1}\right)^p$$

die Höhe des Endschwellenwerts ($y_2$) gemäß: , wobei p eine feste Polynomhöhe ist, wobei für eine quadratische Polynomfunktion p gleich 2 ist;

iii. eine CTF mit einem exponentiellen Übergang von der Höhe des Anfangsschwellenwerts ($y_1$) auf

die Höhe des Endschwellenwerts ($y_2$) gemäß $CTF(x) = e^{\frac{x_2 \cdot In(y_1) - x_1 \cdot In(y_2)}{x_2 - x_1}} \cdot \left(\frac{y_2}{y_1}\right)^{\frac{x}{x_2 - x_1}}$ ;

- Bestimmen einer Quantifizierungszyklusnummer (Cq) der RNR, wobei die Quantifizierungszyklusnummer (Cq) ein quantitatives und/oder qualitatives Analyseergebnis der Wachstumskurve (200) als die Zyklusnummer angibt, die einem Schnittpunkt der Wachstumskurve (200) und der kombinierten Schwellenwertfunktion (CTF) entspricht.

2. Analyseverfahren nach Anspruch 1, wobei:

- die Höhe des Anfangsschwellenwerts (y1) mindestens eine Methode umfasst, die für frühe Reaktionszyklen der RNR angemessen ist; und/oder
- die Höhe des Endschwellenwerts (y2) mindestens eine Methode umfasst, die für späte Reaktionszyklen der RNR angemessen ist.

3. Analyseverfahren nach einem der vorangehenden Ansprüche, wobei:

- die Höhe des Anfangsschwellenwerts (y1) einen Ausgangspunkt ($T_1$) mit einer y-Koordinate oberhalb einer y-Koordinate eines Endpunkts ($T_2$) der Höhe des Endschwellenwerts (y2) aufweist, was zu einem Abstieg der kombinierten Schwellenwertfunktion (CTF) führt.

4. Analyseverfahren nach einem der vorangehenden Ansprüche, wobei die Kombination mindestens eine Schwellenwerthöhe, die für frühe Reaktionszyklen der RNR angemessen ist, und mindestens eine Schwellenwerthöhe, die für späte Reaktionszyklen der RNR angemessen ist, umfasst.

5. Analyseverfahren nach einem der vorangehenden Ansprüche, wobei die Echtzeit-Nukleinsäureamplifikationsreaktion RNR eine Echtzeit-Polymerasekettenreaktion PCR ist.

6. System zum Analysieren einer Echtzeit-Nukleinsäureamplifikationsreaktion RNR eines Analyten, wobei das System umfasst:

- ein Erfassungssystem (130), das dafür ausgelegt ist, eine Intensität einer Fluoreszenzemission des Analyten für jeden Amplifikationsreaktionszyklus der RNR zu ermitteln;
- eine Verarbeitungseinheit (145), die ausgelegt ist zum:

• Erzeugen einer Wachstumskurve (200), welche die Intensität der Fluoreszenzemission über einem Bereich von Reaktionszyklen der RNR angibt,

- Bereitstellen einer kombinierten Schwellenwertfunktion (CTF) über dem RNR-Zyklusbereich, der mindestens zwei verschiedene Schwellenwerthöhen umfasst, wobei die kombinierte Schwellenwertfunktion (CTF) von der Zyklusnummer der RNR abhängt, wobei die kombinierte Schwellenwertfunktion (CTF) von einer Höhe des Anfangsschwellenwerts (y1) auf eine Höhe des Endschwellenwerts (y2) übergeht, wobei die Höhe des Anfangsschwellenwerts (y1) von der Höhe des Endschwellenwerts (y2) verschieden ist,

wobei die Höhe des Anfangsschwellenwerts (y1) B + r x I ist, wobei B eine Grundlinienfunktion ist, wobei die Grundlinie der Anfangsabschnitt der Wachstumskurve ist, der keinen Signalanstieg zeigt, r ein positiver Prozentparameter ist und I eine Achsenabschnittsextrapolation für die Zyklusnummer 0 ist,

wobei die Höhe des Endschwellenwerts (y2) $y(x) = B + p \cdot G = B + p \cdot (S - B)$ ist, wobei B die Grundlinienfunktion ist, S die Sättigungslinie der Wachstumskurve ist, G das Wachstum von der Grundlinie zur Sättigungslinie ist und p ein Parameter zwischen 0 % und 100 % ist, wobei die Höhe des Anfangsschwellenwerts (y1) über der Höhe des Endschwellenwerts (y2) liegt,

- wobei die kombinierte Schwellenwertfunktion ausgewählt ist aus einer Gruppe von drei Funktionen, umfassend:

i. eine CTF mit einem linearen Übergang von der Höhe des Anfangsschwellenwerts ($y_1$) auf die Höhe des Endschwellenwerts ($y_2$) gemäß:
$$CTF(x) = y_1 + (y_2 - y_1) \cdot \frac{x - x_1}{x_2 - x_1}$$
, wobei xl eine Nummer eines frühen Reaktionszyklus gleich 0 oder 1 ist, x2 eine Nummer eines späten Reaktionszyklus größer 30 ist, $y_1$ die Höhe des Anfangsschwellenwerts ist, y2 die Höhe des Endschwellenwerts ist.

ii. eine CTF mit einem polynomischen Übergang von der Höhe des Anfangsschwellenwerts ($y_1$) auf die Höhe des Endschwellenwerts ($y_2$) gemäß:
$$CTF(x) = y_1 + (y_2 - y_1) \cdot \left(\frac{x - x_1}{x_2 - x_1}\right)^p$$
, wobei p eine feste Polynomhöhe ist, wobei für eine quadratische Polynomfunktion p gleich 2 ist;

iii. eine CTF mit einem exponentiellen Übergang von der Höhe des Anfangsschwellenwerts ($y_1$) auf die Höhe des Endschwellenwerts ($y_2$) gemäß $CTF(x) =$
$$e^{\frac{x_2 \cdot In(y_1) - x_1 \cdot In(y_2)}{x_2 - x_1}} \cdot \left(\frac{y_2}{y_1}\right)^{\frac{x}{x_2 - x_1}}$$
; und

• Bestimmen einer Quantifizierungszyklusnummer (Cq) der RNR, wobei die Quantifizierungszyklusnummer (Cq) ein quantitatives und/oder qualitatives Analyseergebnis der Wachstumskurve (200) als die Zyklusnummer angibt, die einem Schnittpunkt der Wachstumskurve (200) und der kombinierten Schwellenwertfunktion (CTF) entspricht.

**7.** System nach Anspruch 6, wobei die kombinierte Schwellenwertfunktion (CTF) von der Verarbeitungseinheit durch Lesen von Daten und/oder (mindestens einem) Befehl, durch den bzw. durch die die kombinierte Schwellenwertfunktion (CTF) beschrieben wird, und/oder durch Berechnen der kombinierten Schwellenwertfunktion (CTF) bereitgestellt wird.

**8.** System nach Anspruch 6 oder 7, wobei die Echtzeit-Nukleinsäureamplifikationsreaktion RNR eine Echtzeit-Polymerasekettenreaktion PCR ist.

**9.** Analyseverfahren für eine Echtzeit-Nukleinsäureamplifikationsreaktion, RNR, wobei das Verfahren umfasst:

- Ermitteln einer Intensität einer Fluoreszenzemission eines Analyten für jeden Amplifikationsreaktionszyklus der RNR;
- Erzeugen einer Wachstumskurve (200), welche die Intensität der Fluoreszenzemission über einem RNR-Zyklusbereich angibt,
- Berechnen einer kombinierten Schwellenwertfunktion (CTF) über dem RNR-Zyklusbereich, der mindestens zwei verschiedene Schwellenwerthöhen umfasst, wobei die kombinierte Schwellenwertfunktion (CTF) von der Zyklusnummer der RNR abhängt, wobei die kombinierte Schwellenwertfunktion (CTF) von einer Höhe des Anfangsschwellenwerts (y1) auf eine Höhe des Endschwellenwerts (y2) übergeht, wobei die Höhe des Anfangsschwellenwerts (y1) von der Höhe des Endschwellenwerts (y2) verschieden ist,

wobei die Höhe des Anfangsschwellenwerts (y1) B + r x I ist, wobei B eine Grundlinienfunktion ist, wobei die Grundlinie der Anfangsabschnitt der Wachstumskurve ist, der keinen Signalanstieg zeigt, r ein positiver Prozentparameter ist und I eine Achsenabschnittsextrapolation auf die Zyklusnummer 0 ist,

wobei die Höhe des Endschwellenwerts (y2) $y(x) = B + p \cdot G = B + p \cdot (S - B)$ ist, wobei B die Grundlinienfunktion ist, S die Sättigungslinie der Wachstumskurve ist, G das Wachstum von der Grundlinie zur Sättigungslinie ist und p ein Parameter zwischen 0 % und 100 % ist,

wobei die Höhe des Anfangsschwellenwerts (y1) unter der Höhe des Endschwellenwerts (y2) liegt,

wobei die kombinierte Schwellenwertfunktion ausgewählt ist aus einer Gruppe von drei Funktionen, umfassend:

i. eine CTF mit einem linearen Übergang von der Höhe des Anfangsschwellenwerts ($y_1$) auf die Höhe des Endschwellenwerts ($y_2$) gemäß:

$$CTF(x) = y_1 + (y_2 - y_1) \cdot \frac{x - x_1}{x_2 - x_1}$$

, wobei x1 eine Nummer eines frühen Reaktionszyklus gleich 0 oder 1 ist, x2 eine Nummer eines späten Reaktionszyklus größer 30 ist, $y_1$ die Höhe des Anfangsschwellenwerts ist, y2 die Höhe des Endschwellenwerts ist.

ii. eine CTF mit einem polynomischen Übergang von der Höhe des Anfangsschwellenwerts ($y_1$) auf die Höhe des Endschwellenwerts ($y_2$) gemäß:

$$CTF(x) = y_1 + (y_2 - y_1) \cdot \left(\frac{x - x_1}{x_2 - x_1}\right)^p$$

, wobei p eine feste Polynomhöhe ist, wobei für eine quadratische Polynomfunktion p gleich 2 ist;

iii. eine CTF mit einem exponentiellen Übergang von der Höhe des Anfangsschwellenwerts ($y_1$) auf die Höhe des Endschwellenwerts ($y_2$) gemäß $CTF(x) =$

$$e^{\frac{x_2 \cdot In(y_1) - x_1 \cdot In(y_2)}{x_2 - x_1}} \cdot \left(\frac{y_2}{y_1}\right)^{\frac{x}{x_2 - x_1}}$$ ;

- Bestimmen einer Quantifizierungszyklusnummer (Cq) der RNR, wobei die Quantifizierungszyklusnummer (Cq) ein quantitatives und/oder qualitatives Analyseergebnis der Wachstumskurve (200) als die Zyklusnummer angibt, die einem Schnittpunkt der Wachstumskurve (200) und der kombinierten Schwellenwertfunktion (CTF) entspricht.

10. System zum Analysieren einer Echtzeit-Nukleinsäureamplifikationsreaktion RNR eines Analyten, wobei das System umfasst:

- ein Erfassungssystem (130), das dafür ausgelegt ist, eine Intensität einer Fluoreszenzemission des Analyten für jeden Amplifikationsreaktionszyklus der RNR zu ermitteln;
- eine Verarbeitungseinheit (145), die ausgelegt ist zum:

• Erzeugen einer Wachstumskurve (200), welche die Intensität der Fluoreszenzemission über einem Bereich von Reaktionszyklen der RNR angibt,

- Bereitstellen einer kombinierten Schwellenwertfunktion (CTF) über dem RNR-Zyklusbereich, der mindestens zwei verschiedene Schwellenwerthöhen umfasst, wobei die kombinierte Schwellenwertfunktion (CTF) von der Zyklusnummer der RNR abhängt, wobei die kombinierte Schwellenwertfunktion (CTF) von einer Höhe des Anfangsschwellenwerts (y1) auf eine Höhe des Endschwellenwerts (y2) übergeht, wobei die Höhe des Anfangsschwellenwerts (y1) von der Höhe des Endschwellenwerts (y2) verschieden ist,

wobei die Höhe des Anfangsschwellenwerts (y1) B + r x I ist, wobei B eine Grundlinienfunktion ist, wobei die Grundlinie der Anfangsabschnitt der Wachstumskurve ist, der keinen Signalanstieg zeigt, r ein positiver Prozentparameter ist und I eine Achsenabschnittsextrapolation für die Zyklusnummer 0 ist,

wobei die Höhe des Endschwellenwerts (y2) $y(x) = B + p \cdot G = B + p \cdot (S - B)$ ist, wobei B die Grundlinienfunktion ist, S die Sättigungslinie der Wachstumskurve ist, G das Wachstum von der Grundlinie zur Sättigungslinie ist und p ein Parameter zwischen 0 % und 100 % ist,

wobei die Höhe des Anfangsschwellenwerts (y1) unter der Höhe des Endschwellenwerts (y2) liegt,

- wobei die kombinierte Schwellenwertfunktion ausgewählt ist aus einer Gruppe von drei Funktionen, umfassend:

i. eine CTF mit einem linearen Übergang von der Höhe des Anfangsschwellenwerts ($y_1$) auf die Höhe des

$$CTF(x) = y_1 + (y_2 - y_1) \cdot \frac{x-x_1}{x_2-x_1}$$

Endschwellenwerts ($y_2$) gemäß: , wobei x1 eine Nummer eines frühen Reaktionszyklus gleich 0 oder 1 ist, x2 eine Nummer eines späten Reaktionszyklus größer 30 ist, $y_1$ die Höhe des Anfangsschwellenwerts ist, y2 die Höhe des Endschwellenwerts ist.

ii. eine CTF mit einem polynomischen Übergang von der Höhe des Anfangsschwellenwerts ($y_1$) auf die

$$CTF(x) = y_1 + (y_2 - y_1) \cdot \left(\frac{x-x_1}{x_2-x_1}\right)^p$$

Höhe des Endschwellenwerts ($y_2$) gemäß: , wobei p eine feste Polynomhöhe ist, wobei für eine quadratische Polynomfunktion p gleich 2 ist;

iii. eine CTF mit einem exponentiellen Übergang von der Höhe des Anfangsschwellenwerts ($y_1$) auf die

Höhe des Endschwellenwerts ($y_2$) gemäß $CTF(x) =$ $e^{\frac{x_2 \cdot In(y_1) - x_1 \cdot In(y_2)}{x_2-x_1}} \cdot \left(\frac{y_2}{y_1}\right)^{\frac{x}{x_2-x_1}}$ ; und

• Bestimmen einer Quantifizierungszyklusnummer (Cq) der RNR, wobei die Quantifizierungszyklusnummer (Cq) ein quantitatives und/oder qualitatives Analyseergebnis der Wachstumskurve (200) als die Zyklusnummer angibt, die einem Schnittpunkt der Wachstumskurve (200) und der kombinierten Schwellenwertfunktion (CTF) entspricht.

## Revendications

1. Procédé d'analyse pour une réaction d'amplification d'acide nucléique RNR en temps réel, le procédé comprenant :

- l'acquisition d'une intensité d'une émission de fluorescence d'un analyte pour chaque cycle de réaction d'amplification de la RNR ;
- la création d'une courbe de croissance (200) indicatrice de l'intensité de l'émission de fluorescence sur une plage de cycle de RNR,
- le calcul d'une fonction de seuil combinée (CTF) sur la plage de cycle de RNR comprenant au moins deux niveaux de seuil différents, où la fonction de seuil combinée (CTF) dépend du numéro de cycle de la RNR, la fonction de seuil combinée (CTF) passant d'un niveau de seuil initial (y1) à un niveau de seuil final (y2), le niveau de seuil initial (y1) étant différent du niveau de seuil final (y2),

le niveau de seuil initial (y1) étant B + r x I, où B est une fonction de d'une ligne de base, la ligne de base étant la partie initiale de la courbe de croissance qui ne présente aucune augmentation de signal, r est un paramètre de pourcentage positif, et I est une extrapolation d'ordonnée pour le numéro de cycle 0, le niveau de seuil final (y2) étant égal à y(x) = B + p · G = B + p · (S - B), où B est la fonction de ligne de base, S est la ligne de saturation de la courbe de croissance, G est la croissance à partir de la ligne de base jusqu'à la ligne de saturation et p est un paramètre entre 0 % et 100 %, où le niveau de seuil initial (y1) est au-dessus du niveau de seuil final (y2), où la fonction de seuil combinée est choisie dans un groupe de trois fonctions comprenant :

i. une CTF avec une transition linéaire à partir du niveau de seuil initial (y1) jusqu'au niveau de seuil final (y2) selon : CTF(x) = $y_1$ + ($y_2$ - $y_1$)) · $\frac{x-x_1}{x_2-x_1}$ où x1 est un numéro de cycle d'une réaction antérieur égal à 0 ou à 1, x2 est un numéro de cycle de réaction postérieur supérieur à 30, y1 est le niveau de seuil initial, y2 est le niveau de seuil final,

ii. une CTF avec une transition polynomiale à partir du niveau de seuil initial (y1) jusqu'au niveau de seuil final (y2) selon : CTF(x) = $y_1$ + ($y_2 -$ $y_1$) $\cdot$ $\left(\frac{x-x_1}{x_2-x_1}\right)^p$ , où p est un ordre polynomial fixe, où p est égal à 2 pour une fonction polynomiale quadratique ;

iii. une CTF avec une transition exponentielle à partir du niveau de seuil initial (y1) jusqu'au niveau de seuil final (y2) selon

$$CTF(x) = e^{\frac{x_2 \cdot ln(y_1) - x_1 \cdot ln(y_2)}{x_2 - x_1}} \cdot \left(\frac{y_2}{y_1}\right)^{\frac{x}{x_2 - x_1}} ;$$

- la détermination d'un numéro de cycle de quantification (Cq) de la RNR, le numéro de cycle de quantification (Cq) étant indicateur d'un résultat d'analyse quantitative et/ou qualitative de la courbe de croissance (200) lorsque le numéro de cycle correspond à une intersection de la courbe de croissance (200) avec la fonction de seuil combinée (CTF).

2. Procédé d'analyse selon la revendication 1, dans lequel :

- le niveau de seuil initial (y1) comprend au moins un procédé adéquat pour des cycles de réaction antérieurs de la RNR ; et/ou
- le niveau de seuil final (y2) comprend au moins un procédé adéquat pour des cycles de réaction ultérieurs de la RNR.

3. Procédé d'analyse selon l'une quelconque des revendications précédentes, dans lequel :

- le niveau de seuil initial (y1) possède un point de départ ($T_1$) avec une coordonnée y au-dessus d'une coordonnée y d'un point final ($T_2$) du niveau de seuil final (y2) résultant d'une fonction de seuil combinée (CTF) décroissante.

4. Procédé d'analyse selon l'une quelconque des revendications précédentes, dans lequel la combinaison comprend au moins un niveau de seuil adéquat pour des cycles de réaction antérieurs de la RNR et au moins un niveau de seuil adéquat pour des cycles ultérieurs de la RNR.

5. Procédé d'analyse selon l'une quelconque des revendications précédentes, dans lequel la réaction d'amplification d'acide nucléique RNR en temps réel est une réaction en chaîne de polymérase PCR en temps réel.

6. Système d'analyse d'une réaction d'amplification d'acide nucléique RNR en temps réel d'un analyte, ledit système comprenant :

- un système de détection (130) conçu pour acquérir une intensité d'une émission de fluorescence de l'analyte pour chaque cycle de réaction d'amplification de la RNR;
- une unité de traitement (145) conçue pour :

• créer une courbe de croissance (200) indicatrice de l'intensité de l'émission de fluorescence sur une plage de cycles de réaction de la RNR ;

- fournir une fonction de seuil combinée (CTF) sur la plage de cycle de RNR comprenant au moins deux niveaux de seuil différents, où la fonction de seuil combinée (CTF) dépend du numéro de cycle de la RNR, la fonction de seuil combinée (CTF) passant d'un niveau de seuil initial (y1) à un niveau de seuil final (y2), le niveau de seuil initial (y1) étant différent du niveau de seuil final (y2),

le niveau de seuil **initial (y1)** étant égal à B + r x I, où B est une fonction de la ligne de base, la ligne de base étant la partie initiale de la courbe de croissance qui ne présente aucune augmentation de signal, r est un paramètre de pourcentage positif, et I est une extrapolation d'ordonnée pour le numéro de cycle 0,

le niveau de seuil **final (y2)** étant égal à y(x) = B + p · G = B + p ·(S - B), où B est la fonction de ligne de base, S est la ligne de saturation de la courbe de croissance, G est la croissance à partir de la ligne de base jusqu'à la ligne de saturation et p est un paramètre entre 0 % et 100 %, où le niveau de seuil initial (y1) est au-dessus du niveau de seuil final (y2), où la fonction de seuil combinée est choisie dans un groupe de trois fonctions comprenant :

i. une CTF avec une transition linéaire à partir du niveau de seuil initial (y1) jusqu'au niveau de seuil final (y2) selon : $CTF(x) = y_1 + (y_2 - y_1) \cdot \dfrac{x - x_1}{x_2 - x_1}$ où x1 est un numéro de cycle d'une réaction antérieur égal à 0 ou à 1, x2 est un numéro de cycle de réaction postérieur supérieur à 30, y1 est le niveau de seuil initial, y2 est le niveau de seuil final,

ii. une CTF avec une transition polynomiale à partir du niveau de seuil initial (y1) jusqu'au niveau de seuil final (y2) selon : $CTF(x) = y_1 + (y_2 - y_1) \cdot \left(\dfrac{x - x_1}{x_2 - x_1}\right)^p$ , où p est un ordre polynomial fixe, où p est égal à 2 pour une fonction polynomiale quadratique ;

iii. une CTF avec une transition exponentielle à partir du niveau de seuil initial (y1) jusqu'au niveau de seuil final (y2) selon $CTF(x) = e^{\frac{x_2 \cdot ln(y_1) - x_1 \cdot ln(y_2)}{x_2 - x_1}} \cdot \left(\dfrac{y_2}{y_1}\right)^{\frac{x}{x_2 - x_1}}$ ; et

- déterminer un numéro de cycle de quantification (Cq) de la RNR, le numéro de cycle de quantification (Cq) étant indicateur d'un résultat d'analyse quantitative et/ou qualitative de la courbe de croissance (200) pour un numéro de cycle correspondant à une intersection de la courbe de croissance (200) avec la fonction de seuil combinée (CTF).

**7.** Système selon la revendication 6, dans lequel la fonction de seuil combinée (CTF) est fournie par l'unité de traitement en lisant des données et/ou une(des) instruction(s) à partir d'une mémoire électronique du système qui est descriptrice de la fonction de seuil combinée (CTF) et/ou en calculant la fonction de seuil combinée (CTF).

**8.** Système selon la revendication 6 ou la revendication 7, dans lequel la réaction d'amplification d'acide nucléique RNR en temps réel est une réaction de chaîne polymérase PCR en temps réel.

**9.** Procédé d'analyse d'une réaction d'amplification d'acide nucléique RNR en temps réel, le procédé comprenant :

- l'acquisition d'une intensité d'une émission de fluorescence d'un analyte pour chaque cycle de réaction d'amplification de la RNR ;
- la création d'une courbe de croissance (200) indicatrice de l'intensité de l'émission de fluorescence sur une plage de cycle de RNR,
- le calcul d'une fonction de seuil combinée (CTF) sur la plage de cycle de RNR comprenant au moins deux niveaux de seuil différents, où la fonction de seuil combinée (CTF) dépend du numéro de cycle de la RNR, la fonction de seuil combinée (CTF) passant d'un niveau de seuil initial (y1) à un niveau de seuil final (y2), le niveau de seuil initial -y1) étant différent du niveau de seuil final (y2),

le niveau de seuil initial (y1) étant égal à B + r x I, où B est une fonction de la ligne de base, la ligne de base étant la partie initiale de la courbe de croissance qui ne présente aucune augmentation de signal, r est un paramètre de pourcentage positif, et I est une extrapolation d'ordonnée pour le numéro de cycle 0, le niveau de seuil final (y2) étant égal à y(x) = B + p · G = B + p · (S - B), où B est la fonction de la ligne de base, S est la ligne de saturation de la courbe de croissance, G est la croissance à partir de la ligne de base jusqu'à la ligne de saturation et p est un paramètre entre 0 % et 100 %, où le niveau de seuil initial (y1) est en-dessous du niveau de seuil final (y2), où la fonction de seuil combinée est choisie dans un groupe de trois fonctions comprenant :

**i.** une CTF avec une transition linéaire à partir du niveau de seuil initial $(y_1)$ jusqu'au niveau de seuil final $(y_2)$ selon : $\text{CTF}(x) = y_1 + (y_2 - y_1) \cdot \dfrac{x - x_1}{x_2 - x_1}$ où x1 est un numéro de cycle d'une réaction antérieur égal à 0 ou à 1, x2 est un numéro de cycle de réaction postérieur supérieur à 30, y1 est le niveau de seuil initial, y2 est le niveau de seuil final y2,

**ii.** une CTF avec une transition polynomiale à partir du niveau de seuil initial $(y_1)$ jusqu'au niveau de seuil final $(y_2)$ selon : $\text{CTF}(x) = y_1 + (y_2 - y_1) \cdot \left(\dfrac{x - x_1}{x_2 - x_1}\right)^p$ , où p est un ordre polynomial fixe, où p est égal à 2 pour une fonction polynomiale quadratique ;

**iii.** une CTF avec une transition exponentielle à partir du niveau de seuil initial $(y_1)$ jusqu'au niveau de seuil final $(y_2)$ selon $\text{CTF}(x) = e^{\frac{x_2 \cdot ln(y_1) - x_1 \cdot ln(y_2)}{x_2 - x_1}} \cdot \left(\dfrac{y_2}{y_1}\right)^{\frac{x}{x_2 - x_1}}$ ;

- la détermination d'un numéro de cycle de quantification (Cq) de RNR, le numéro de cycle de quantification (Cq) étant indicateur d'un résultat d'analyse quantitative et/ou qualitative de la courbe de croissance (200) pour un numéro de cycle correspondant à une intersection de la courbe de croissance (200) avec la fonction de seuil combinée (CTF).

**10.** Système d'analyse d'une réaction d'amplification d'acide nucléique RNR en temps réel d'un analyte, ledit système comprenant :

- un système de détection (130) conçu pour acquérir une intensité d'une émission de fluorescence de l'analyte pour chaque cycle de réaction d'amplification de la RNR;
- une unité de traitement (145) conçue pour :

• créer une courbe de croissance (200) indicatrice de l'intensité de l'émission de fluorescence sur une plage de cycles de réaction de la RNR ;

- fournir une fonction de seuil combinée (CTF) sur la plage de cycle de RNR comprenant au moins deux niveaux de seuil différents, où la fonction de seuil combinée (CTF) dépend du numéro de cycle de la RNR, la fonction de seuil combinée (CTF) passant d'un niveau de seuil initial (y1) à un niveau de seuil final (y2), le niveau de seuil initial (y1) étant différent du niveau de seuil final (y2),

le niveau de seuil initial (y1) étant égal à B + r x I, où B est une fonction de la ligne de base, la ligne de base étant la partie initiale de la courbe de croissance qui ne présente aucune augmentation de signal, r est un paramètre de pourcentage positif, et I est une extrapolation d'ordonnée pour le numéro de cycle 0, le niveau de seuil final (y2) étant égal à y(x) = B + p · G = B + p · (S - B), où B est la fonction de la ligne de base, S est la ligne de saturation de la courbe de croissance, G est la croissance à partir de la ligne de base jusqu'à la ligne de saturation et p est un paramètre entre 0 % et 100 %, où le niveau de seuil initial (y1) est **en-dessous** du niveau de seuil final (y2), où la fonction de seuil combinée est choisie dans un groupe de trois fonctions comprenant :

i. une CTF avec une transition linéaire à partir du niveau de seuil initial ($y_1$) jusqu'au niveau de seuil final ($y_2$) selon :

$$CTF(x) = y_1 + (y_2 - y_1) \cdot {}^{\frac{x-x_1}{x_2-x_1}}$$

où x1 est un numéro de cycle d'une réaction antérieur égal à 0 ou à 1, x2 est un numéro de cycle de réaction postérieur supérieur à 30, y1 est le niveau de seuil initial, y2 est le niveau de seuil final y2,

ii. une CTF avec une transition polynomiale à partir du niveau de seuil initial ($y_1$) jusqu'au niveau de seuil final ($y_2$) selon :

$$CTF(x) = y_1 + (y_2 - y_1) \cdot \left(\frac{x-x_1}{x_2-x_1}\right)^p$$

, où p est un ordre polynomial fixe, où p est égal à 2 pour une fonction polynomiale quadratique ;

iii. une CTF avec une transition exponentielle à partir du niveau de seuil initial ($y_1$) jusqu'au niveau de seuil final ($y_2$) selon

$$CTF(x) = e^{\frac{x_2 \cdot ln(y_1) - x_1 \cdot ln(y_2)}{x_2-x_1}} \cdot \left(\frac{y_2}{y_1}\right)^{\frac{x}{x_2-x_1}}$$

;

- déterminer un numéro de cycle de quantification (Cq) de RNR, le numéro de cycle de quantification (Cq) étant indicateur d'un résultat d'analyse quantitative et/ou qualitative de la courbe de croissance (200) pour un numéro de cycle correspondant à une intersection de la courbe de croissance (200) avec la fonction de seuil combinée (CTF).

# Fig. 1

# Fig. 2

# Fig. 3A

# Fig. 3B

$$\log_2 10^{7-1} = 19.93 \cong 20$$

## Fig. 4

$$\log_2 10^{7-1} = 19.93 \cong 20$$

## Fig. 5

# Fig. 6

# Fig. 7

# Fig. 8

**Transition Functions**

# Fig. 9

**Growth Curves of Dilution Series**

**Fig. 10** Thresholds of Dilution Series

## Fig. 11

**Cq as Function of Concentration**

## Fig. 12

**Growth Curves of Interferences**

## Fig. 13

**Thresholds of Interferences**

## Fig. 14

**Cq as Function of Interferent**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2719770 A1
- US 7788039 B2
- EP 1798652 B1
- EP 1798542 A1

**Non-patent literature cited in the description**

- **THOMAS D SCHMITTGEN et al.** Analyzing real-time PCR data by the comparative CT method. *Nature Protocols,* 2008, vol. 3, 1101-1108